(19) Europäisches Patentamt
European Patent Office
Office européen des brevets

(11) Veröffentlichungsnummer : **0 010 235**
**B1**

(12) # EUROPÄISCHE PATENTSCHRIFT

(45) Veröffentlichungstag der Patentschrift : **23.12.81**

(21) Anmeldenummer : **79103814.4**

(22) Anmeldetag : **05.10.79**

(51) Int. Cl.³ : **C 07 C 47/21**, C 07 C 45/51// C07C43/15, C07C41/28

(54) **2-Propylpent-4-en 1-al und Verfahren zu dessen Herstellung.**

(30) Priorität : **13.10.78 DE 2844635**

(43) Veröffentlichungstag der Anmeldung : **30.04.80 (Patentblatt 80/09)**

(45) Bekanntmachung des Hinweises auf die Patenterteilung : **23.12.81 Patentblatt 81/51**

(84) Benannte Vertragsstaaten : **BE CH FR GB IT LU SE**

(56) Entgegenhaltungen :
**DE - B - 2517 447**

**JOURNAL OF THE AMERICAN CHEMICAL SOCIETY, Vol. 81, 5. Juli 1959, Eaton, GB K.C. BRANNOCK : « Preparation of substituted 4-Pentenals », Seiten 3379-3383**

(73) Patentinhaber : **Ruhrchemie Aktiengesellschaft**
**Bruchstrasse 219**
**D-4200 Oberhausen 13 (DE)**

(72) Erfinder : **Weber, Jürgen, Dr. Dipl.-Chem**
**Bunsenstrasse 17**
**D-4200 Oberhausen 13 (DE)**
Erfinder : **Bernhagen, Wolfgang, Dr. Dipl.-Chem.**
**Witthausstrasse 19**
**D-4330 Mülheim/Ruhr (DE)**
Erfinder : **Springer, Helmut**
**Drostenkampstrasse 24**
**D-4200 Oberhausen 13 (DE)**

(74) Vertreter : **Reichelt, Karl-Heinz, Dr.**
**m. Br. Ruhrchemie Aktiengesellschaft Abt. PLD Postfach 13 01 60**
**D-4200 Oberhausen 13 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

**EP 0 010 235 B1**

2-Propylpent-4-en-1-al und Verfahren zu dessen Herstellung

Die Erfindung betrifft den ungesättigten Aldehyd 2-Propylpent-4-en-1-al der Formel

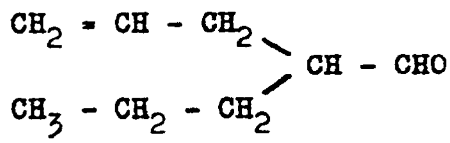

Pent-4-en-1-al und verschiedene Substitutionsprodukte dieses ungesättigten Aldehyds sind aus der Literatur bekannt.

In der DE-AS 25 17 447 ist die Herstellung von Pent-4-en-1-al aus einem Acetaldehydacetal, das zumindest einen Allylalkoholrest enthält, beschrieben. Nach der bekannten Arbeitsweise wird das Acetal bei 350 bis 450 °C in der Gasphase über einen oberflächenaktiven Kontakt geleitet. Unter den Reaktionsbedingungen spaltet sich ein Mol Allylalkohol ab. Der dabei entstehende doppelt ungesättigte Äther lagert sich zum Pent-4-en-1-al um.

Die Herstellung substituierter Pent-4-en-1-ale ist Gegenstand einer Veröffentlichung von Kent C. Brannock in J. Am. Chem. Soc. 81 (1959), 3 382. Als Ausgangsstoffe werden Aldehyddiallylacetale verwendet, aus denen unter der katalytischen Einwirkung von Säure ein Mol Allylalkohol abgespalten wird. Es entstehen Allylalkenyläther, die sich ohne vorherige Isolierung in die substituierten 4-Pentenale umlagern lassen.

2-Propyl-pent-4-en-1-al, eine bisher in der Literatur nicht beschriebene Verbindung, ist ein Duftstoff mit einer Geruchsnuance, die sich von anderen, auf dem Markt befindlichen Duftstoffen unterscheidet. Sie kann darüber hinaus als Zwischenprodukt zur Herstellung anderer organischer Verbindungen, z.B. 2.2-Dipropylethanol, 2.2-Dipropyl-ethylamin, 2-Propyl-valeraldehyd, 2-Propyl-hexan-1,6-dial Verwendung finden. 2-Propyl-pent-4-en-1-al siedet bei 161 °C.

Die Herstellung von 2-Propyl-pent-4-en-1-al erfolgt erfindungsgemäß aus n-Valeraldehyd, der mit 2 Mol Allylalkohol zum entsprechenden Diacetal umgesetzt wird. Die Acetalbildung nimmt man vorzugsweise in einem Lösungsmittel, z.B. Cyclohexan, n-Hexan, n-Hexen und in Gegenwart eines sauren Katalysators, z.B. p-Toluolsulfonsäure, vor.

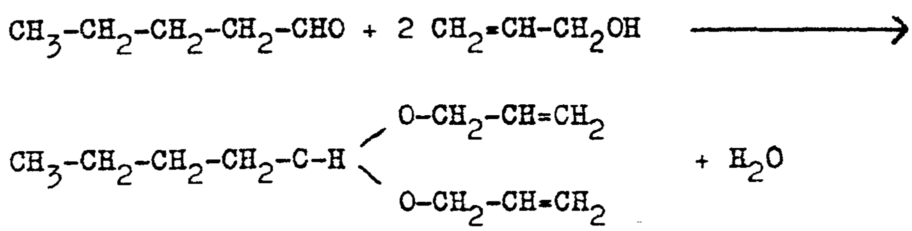

Anschließend wird das Rohacetal destilliert und bei Temperaturen von 120 bis 190 °C thermisch gespalten, wobei sich nach der folgenden Reaktionsgleichung Allyl-1-pentenyl-äther bildet:

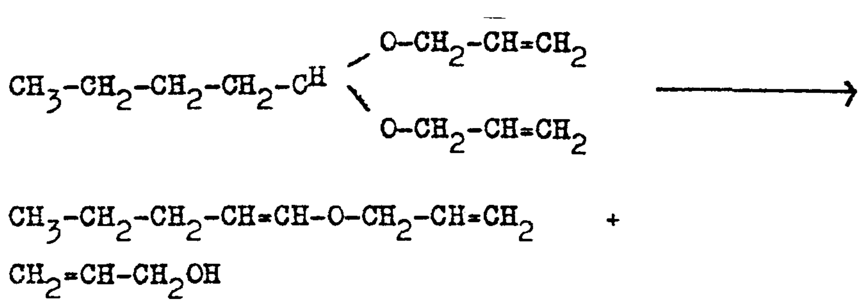

Der ungesättigte Äther schließlich wird bei 250 bis 350 °C thermisch zu der erfindungsgemäßen Verbindung umgelagert.

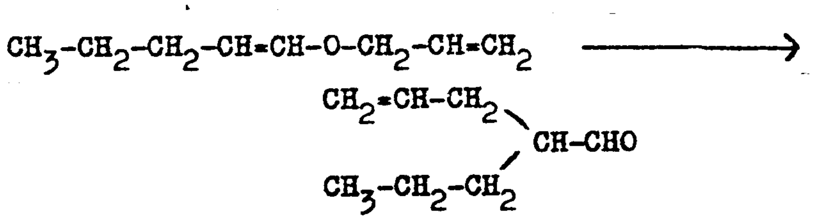

Spaltung des Acetals und Umlagerung können zumindest teilweise nebeneinander verlaufen. Das hat zur Folge, daß das Destillationsprodukt des Rohacetals nicht nur den ungesättigten Äther, sondern bereits das Endprodukt, den ungesättigten Aldehyd, enthält.

Gegenüber den bekannten Herstellungsverfahren zur Gewinnung von Pent-4-en-1-al beziehungsweise den substituierten Pentenalen hat die neue Arbeitsweise den Vorteil, daß die 2-Propyl-pent-4-en-1-ale in einem nichtkatalytischen rein thermischen Prozeß mit hoher Ausbeute hergestellt werden können.

In dem folgenden Beispiel wird die Herstellung des neuen, ungesättigten Aldehyds beschrieben.

In einem mit Rührer, Innenthermometer, Rückflußkühler und Wasserabscheider versehenen 6 1-Rundkolben werden 1 160 g Allylalkohol (20 Mol), 860 g n-Valeraldehyd (10 Mol), 1 200 g n-Hexan und 1 g p-Toluolsulfonsäure auf Rückflußtemperatur erhitzt. Das anfallende Reaktionswasser wird kontinuierlich über den Wasserabscheider ausgekreist. Nach 6 Stunden erreicht die Sumpftemperatur ein Maximum von 77 °C, worauf die Reaktion abgebrochen wird. Die Ausbeute an n-Pentanal-diallylacetal beträgt etwa 72 % der Theorie.

Das Rohacetal wird in einer 1 m Kolonne mit 24 theoretischen Böden so destilliert, daß zunächst bei einer Kopftemperatur von 66 °C und einer Sumpftemperatur von 120 °C (bei Normaldruck) der größte Teil des n-Hexans als 1. Fraktion übergeht. Anschließend stellt man bei einem Druck von 0,133 bar (100 Torr) eine Kopftemperatur von 145 °C und eine Sumpftemperatur von 182 °C ein.

Unter diesen Reaktionsbedingungen wird das Vollacetal nahezu quantitativ gespalten, es bildet sich hauptsächlich der zweifach ungesättigte Allyl-1-pentenyläther, der zum Teil zu 2-Propyl-pent-4-en-1-al weiterreagiert. Die 2. Fraktion hat folgende gaschromatographisch bestimmte Zusammensetzung :

2,1 n-Hexan
6,1 n-Valeraldehyd
30,6 Allylalkohol
44,2 Allyl-1-pentenyläther
11,7 isomere Äther
5,3 2-Propyl-pent-4-en-1-al

Die Ausbeute — gerechnet als Summe Allyl-1-pentenyl-äther, isomere Äther und 2-Propyl-pent-4-en-1-al — beträgt etwa 69 % der Theorie, bezogen auf eingesetzten n-Valeraldehyd beziehungsweise etwa 96 % der Theorie, bezogen auf eingesetztes Vollacetal.

Die wie oben beschrieben erhaltene 2. Fraktion wird in einem elektrisch beheizten Rohr, das mit einem Intensivkühler und einem Auffangkolben versehen ist, thermisch behandelt. Das Rohr ist mit Raschigringen der Abmessungen 5 × 4 mm gefüllt ; der Rohrinhalt beträgt 380 ml. Mit Hilfe einer Dosierpumpe werden 570 ml Flüssigkeit/h entsprechend einer Raumgeschwindigkeit von 1,5 bei 300 °C über das Rohr gegeben.

Aus 1 410 g Roh-Allyl-1-pentenyläther erhält man 1 375 g Roh-2-Propyl-pent-4-en-1-al.

Durch fraktionierte Destillation werden 821 g, entsprechend einer Ausbeute von 95 %, eines 99,5 %igen 2-Propyl-pent-4-en-1-al gewonnen, entsprechend einer Gesamtausbeute von 65 %, bezogen auf eingesetzten n-Valeraldehyd. Durch Wiedereinsatz von nicht umgesetzten Ausgangsmaterialien läßt sich die Ausbeute noch wesentlich verbessern.

**Ansprüche**

1. 2-Propyl-pent-4-en-1-al der Formel

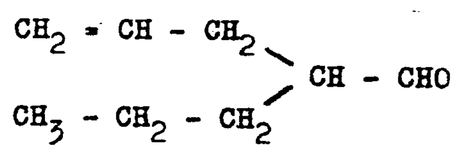

2. Verfahren zur Herstellung von 2-Propyl-pent-4-en-1-al, dadurch gekennzeichnet, daß Valeraldehyd-diallylacetal bei Temperaturen von 120 bis 190 °C thermisch behandelt und der entstandene ungesättigte Äther anschließend bei 250 bis 350 °C umgelagert wird.

**Claims**

1. 2-Propyl-pent-4-en-1-al, which has the formula

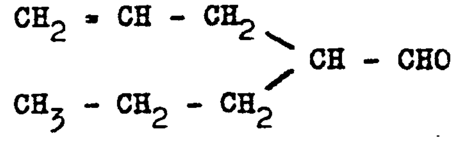

2. A process of preparing 2-propyl-pent-4-en-1-al, comprising subjecting valeraldehyde-diallylacetal to a temperature of 120 to 190 °C to form an unsaturated ether and subjecting the ether to a temperature of 250 to 350 °C to effect molecular rearrangement of the ether to form 2-propyl-pent-4-en-1-al.

**Revendications**

1. 2-propyl-pent-4-ène-1-al de formule

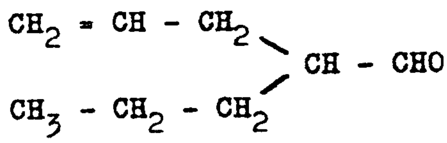

2. Procédé pour la préparation du 2-propyl-pent-4-ène-1-al, caractérisé en ce que l'on traite thermiquement le diallylacétal de valéraldéhyde à des températures de 120 à 190 °C et on transpose ensuite l'éther insaturé qui se forme à 250-350 °C.